# EUROPEAN PATENT APPLICATION

(11) **EP 3 604 554 A1**
(43) Date of publication of application: **05.02.2020**
(21) Application number: 18186997.5
(22) Date of filing: 02.08.2018
(51) Int. Cl.: C12Q 1/6811, C12N 15/115, G01N 33/53

(54) **A METHOD OF IDENTIFYING OR PRODUCING AN APTAMER FOR A DENATURED PEPTIDE OR PROTEIN**

(71) Applicant: Rheinische Friedrich-Wilhelms-Universität Bonn, 53113 Bonn (DE)
(72) Inventor: Mayer, Günter, 53123 Bonn (DE); Karnowski, Nora, 53347 Alfter (DE); Hamann, Maren, 50937 Köln (DE); Legen, Tjasa, 53115 Bonn (DE)
(74) Representative: Michalski Hüttermann & Partner Patentanwälte mbB

(57) **Abstract**

The invention relates to a method of identifying or producing an aptamer, the method comprising the steps of: a) Providing a candidate mixture of nucleic acids; b) Contacting the candidate mixture with a target sample; c) Partitioning nucleic acids that bind to the target sample from those that do not bind; d) Amplifying the binding nucleic acids to produce a population of nucleic acids that bind the target sample; e) Single strand displacement of the amplified nucleic acids, f) optionally repeating the steps a) to e), thereby identifying or producing an aptamer, wherein the target sample provides one or more peptides and/or proteins in a denatured form.

## Description

The present invention relates to the technical field of aptamers, particularly to a method of identifying or producing an aptamer.

Aptamers are versatile, single stranded oligonucleotides that are *in vitro*-selected to recognize their target molecule with high affinity and specificity. Aptamers of either single-stranded DNA or RNA that specifically bind to a target ligand are produced by a technique denoted Systematic Evolution of Ligands by Exponential Enrichment (SELEX), also referred to as *in vitro* selection or *in vitro* evolution. The SELEX method is for example described by C. Tuerk and L. Gold "Systematic Evolution of Ligands by Exponential Enrichment: RNA Ligands to bacteriophage T4 DNA polymerase", Science 1990, 249, 505-510. In the SELEX method, a candidate mixture of single stranded nucleic acids having regions of randomized sequence is contacted with a target structure and those nucleic acids having an increased affinity to the target are selected and amplified. After several iterations a nucleic acid with high affinity to the target is obtained.

Traditionally, aptamers were evolved against small molecules or proteins, and subsequently modified to enable application in a variety of *in vitro* assays. Kyung-Mi Song et al. for example describe in "Aptamers and Their Biological Applications", Sensors 2012, 12, 612-631, several analytical and medical applications of aptamers, including an aptamer-based Western blot analysis. An aptamer specific for the His-tag detects a target protein provided with a His-tag using only one aptamer and thus reduces the Western blot procedure, which usually uses primary and secondary antibody binding steps, to one step.

Generally, the success rate of selection procedures toward protein-specific aptamers is quite low, as many proteins escape the selection process. Nucleobase-modified aptamers, bearing additional chemical functional groups or an extended genetic alphabet have been proven to enhance the success rate and the performance of *in vitro* selection. A protocol that combines click chemistry with nucleic acid libraries and *in vitro* selection procedures is termed click-SELEX and is described in WO 2015/049356 A1. The click-SELEX procedure further is described in detail by F. Pfeiffer et al. in "Identification and characterization of nucleobase-modified aptamers by click-SELEX", Nature Protocols, Vol. 13 No.5, 2018, 1153-1180. Click-SELEX makes use of well-established alkyne-modified nucleotide building blocks that are incorporated into the DNA instead of thymidine. These building blocks are subsequently modified with an azide of choice. The thereby-modified DNA is compatible with the conventional steps of the selection procedure and the routinely used amplification processes.

Although aptamers and particularly click-SELEX selected aptamers can by synthesized chemically and can provide reproducible analytical detection, the analytical applications of aptamers however are limited and oftentimes aptamers fail to provide analytical results.

Therefore, it is an object of the present invention to provide a method that allows for the selection of aptamers providing improved recognition of their target molecule in *in vitro* detection methods.

This object is achieved by a method of identifying or producing an aptamer according to claim 1, the use according to claim 13, and aptamers according to claim 15. Advantageous embodiments are the subject of the dependent claims. They may be combined freely unless the context clearly indicates otherwise.

Accordingly, a method of identifying or producing an aptamer is provided, the method comprising the steps of:
a) Providing a candidate mixture of nucleic acids;
b) Contacting the candidate mixture with a target sample;
c) Partitioning nucleic acids that bind to the target sample from those that do not bind;
d) Amplifying the binding nucleic acids to produce a population of nucleic acids that bind the target sample;
e) Single strand displacement of the amplified nucleic acids,
f) optionally repeating the steps a) to e),
thereby identifying or producing an aptamer,
wherein the target sample provides one or more peptides and/or proteins in a denatured form.

The method enables to select aptamers that provide a reliable and reproducible detection of target proteins in *in vitro* detection methods such as Western blotting. The aptamers produced by the method of the invention are able to reliably and reproducibly bind a target protein on a Western blot with high sensitivity and selectivity. The success of a Western blot generally is limited by the specificity of the primary antibody, and the aptamer provides improved specificity compared to usual primary antibodies that often bind to a variety of proteins. The aptamers further allow to be easily modified to incorporate different reporter molecules or tags that allow either for a direct detection on a Western blot, such as by a fluorescent labeling, or a detection via usual secondary antibodies, such as by an antigen labeled aptamer. The method of the invention allows for the production of versatile applicable aptamers that provide highly specific detection on Western blots with high reliability.

As used herein, the term "aptamer" refers to a small single-stranded oligonucleotide that recognizes its target with high specificity and binds to the target with high affinity.

As used herein, the term "candidate mixture" refers to a mixture of nucleic acids of differing sequence, from which to select a desired ligand. The source of a candidate mixture can be from naturally-occurring nucleic acids or fragments thereof, chemically synthesized nucleic acids, enzymatically synthesized nucleic acids or nucleic acids made by a combination of these techniques.

As used herein, the term "ligand" refers to a nucleic acid that binds another molecule, e.g. the target. In a mixture of candidate nucleic acids, a ligand binds with greater affinity than that of the bulk mixture. In a candidate mixture can be comprised more than one ligand for a given target. The ligands can differ from one another in their binding affinities for the target.

As used herein, the term "nucleobase" refers to the nitrogen-containing biological compounds found within the DNA and RNA nucleotides and nucleosides, usually referred to as bases. The primary nucleobases are cytosine (C), guanine (G), adenine (A), thymine (T), and uracil (U). As used herein, the term "nucleotide" refers to the basic building blocks of nucleic acids comprising or consisting of a nucleobase bound, ribose or deoxyribose and at least one phosphate group. If not stated otherwise, the term "nucleic acid" refers to a ribonucleic acid or a deoxyribonucleic acid, single-stranded or double stranded. An aptamer hence can be provided in the form of a single-stranded DNA or RNA molecule.

As used herein, the term "target sample" refers to a material comprising the target proteins or peptides for which an aptamer is desired. A "sample" can be any material, which probably provides the target to be determined, including a sample derived from a biological source. The term sample also refers to extracts or supernatants of biological material, for example cells, cell organelles, or tissues. According to the invention, the target is a peptide or protein in its denatured form. The target sample may be a single peptide or protein or may be an extract containing the target of interest as one of a multitude of peptides or proteins. The target sample particularly may be separated on a Western Blot membrane.

As used herein, the term "amplifying" refers to any process or combination of process steps that increases the amount or number of copies of a molecule such as a nucleic acid. Polymerase chain reaction (PCR) is an exemplary method for amplifying of nucleic acids.

As used herein, the term "partitioning" refers to any process whereby aptamers bound to the target sample can be separated from nucleic acids not bound to the target. The choice of partitioning methods will depend on properties of the target sample and of the nucleic acids that bind to the target sample. The partitioning may include process steps such as purification and washing steps according to principles and properties known to those of ordinary skill in the art.

Peptides are distinguished from proteins on the basis of size. As used herein, the term "peptide" refers to a sequence of amino acids made up of a single chain of amino acids joined by peptide bonds. Generally, peptides contain less than about 50 amino acids, unless otherwise defined. As used herein, the term "protein" refers to large polypeptides containing more than about 50 amino acids.

As used herein, the term "denatured form" refers to a biologically inactive form of a protein, with reduced or without its native conformation and/or biological activity, such as obtained after gel electrophoresis under conditions under which the native three dimensional structure of the protein is disrupted, for example by treatment with SDS.

As used herein, the term "Western blot" refers to an analytical technique used to detect specific proteins in a given sample, such as a cell or tissue homogenate or extract. It utilizes gel electrophoresis to separate denatured proteins by their lengths. The separated proteins are transferred to a membrane, typically nitrocellulose or polyvinylidene fluoride (PVDF), and are detected, usually using antibodies specific for a target protein.

As used herein, the term "click chemistry" refers to bio-orthogonal reactions known to those of ordinary skill in the art. The term "click chemistry" can refer to a 1,3-dipolar cycloaddition. An example of click chemistry is the 1,3-dipolar cycloaddition of an azide to an alkyne, for example using the Cu(I) catalyzed 1,3-dipolar cycloaddition of an azide with an alkyne (CuAAC), or the copper-free strain-promoted azide-alkyne cycloaddition (SPAAC).

As used herein, the term "alkyne" refers to an unsaturated carbon moiety that has a carbon carbon triple bond between two carbon atoms. As used herein, the term "azide" refers to a functional group RN₃. The group R refers to a chemical modification or additional chemical entity that can be introduced into the DNA. For example the group R can be benzyl or indole. As used herein, the term "azide-alkyne chemical group" refers to a reaction product of an alkyne and an azide. The reaction can be an 1,3-dipolar cycloaddition of the azide with the alkyne. Via 1,3-dipolar cycloaddition of an azide with an alkyne the azide-alkyne linkeage can be a triazole linkage.

The method of the invention uses a SELEX or click SELEX procedure using peptides and/or proteins in their denatured form as a target sample. This allows to select aptamers that provide a detection of denatured target proteins or peptides in *in vitro* detection methods such as Western blotting. In preferred embodiments of the method, an aptamer recognising the denatured target peptide or protein is identified or produced. The selected aptamer may specifically recognise denatured proteins. Depending on the binding epitope, selected aptamers alternatively may also be able to bind to the proteins or peptides being not in their denatured form. Such aptamers may also be usable to detect the proteins or peptides in their native form. Being selected against denatured target proteins or peptides, the thus identified or produced aptamers will bind with high reproducibility to their denatured target peptides or proteins in protein labeling or analysis assays. This allows their use as detection molecules for example in Western blot assays. Aptamers therefore can improve the reproducibility of results in research and diagnostics in their application as a detection molecule, compared, for example, to antibodies. This provides a prominent advantage of the method. Aptamers further can be synthesized chemically and thus reduce the costs of protein detection assays.

The target sample comprising denatured peptides and/or proteins may be received from a protein assay or other sources. In preferred embodiments, in step b) the target sample is provided on a Western blot. Presenting the denatured peptides and proteins gel purified as separated bands and transferred onto a filter membrane, facilitates the interaction with the candidate aptamers. Further, being selected under the conditions of a later use, ensures that the aptamers will provide full functionality in their intended application. Providing the target sample on a Western blot thus ensures for the production of aptamers which are suitable as detection molecules in Western blots. The target sample may comprise in a range of 0.01 µg to 2 µg of denatured peptides and/or proteins.

The procedures for providing a Western blot membrane providing the target sample thereon may be performed according to known methods. In a first step of a Western blot electrophoresis is used to separate the proteins in a gel. In a second step the separated proteins are transferred to a membrane. For the electrophoresis a sodium dodecyl sulfate polyacrylamide (SDS-PAGE) gel may be used. Incubation of a target sample in SDS provides a denaturing effect on the peptides and proteins, which may be enforced by heat treatment. The membrane may be a nitrocellulose membrane. Afterwards the membrane may be blocked by incubation in a blocking agent such as bovine serum albumin (BSA). This reduces or prevents non-specific binding. After blocking the membrane may be washed to remove redundant blocking agent.

The method, particularly an on-blot SELEX or on-blot click-SELEX method, can be applied using a single protein, for example a recombinant protein, as the target sample or a complex target, such as cell lysates or fractions thereof like a membrane fraction. In an embodiment, in step b) the target sample is provided as an isolated peptide or protein. An isolated peptide or protein can be provided as a single protein band on a Western blot membrane. In other embodiments, the target sample in step b) is provided as a multitude of peptides or proteins. The multitude of peptides or proteins may be a random mixture or any kind or synthetic or natural mixture of peptides and/or proteins, particularly separated on a Western blot membrane. Preferably, the target sample may be a complex sample, such as a protein sample of a cell, a cell organelle or membrane, a tissue or an organism, particularly a cellular lysate, such as a whole cell lysate, or a fraction of peptides or proteins thereof such as a membrane fraction.

After contacting the candidate mixture with a target sample in step b), in the following step c) the nucleic acids that bind to the target sample are partitioned from nucleic acids that are not binding. The partitioning process is used to separate aptamers bound to the target sample from aptamers not binding to the target. Generally, binding aptamers are recovered from the target, and subjected to purifying and optionally washing before being amplified and optionally used as a candidate mixture in further SELEX cycles. Embodiments wherein the target sample is provided in form of protein lanes separated on a Western blot provide the advantage of a further possibility of partitioning by selecting individual protein bands and the aptamers binding thereto for further processing in SELEX rounds. In embodiments, in step c) a single protein band or a plurality of protein bands on the Western blot membrane are selected for recovery of binding nucleic acids. This selection of specific protein bands allows for a further selection step. The one or more protein bands on the Western blot membrane that are selected for recovery may be simply excised from the membrane.

In embodiments wherein a single protein is separated on a Western blot the excising of the protein band reduces the presence of unspecific aptamers. In embodiments wherein the target sample provides a multitude of peptides or proteins, a variety of selection options are provided, as in each SELEX round one or more protein bands can be chosen for separation. In embodiments, in step c) for an initial number of SELEX rounds a plurality of protein bands on the Western blot membrane are selected for recovery and for a following number of SELEX rounds a single protein band is selected for recovery of binding nucleic acids. For example, for an initial number of five, six or seven rounds of SELEX may be selected against all proteins or a certain fraction, such as membrane proteins, whereas in the last one, two or three rounds may be selected against a single protein band.

In the following process steps, the nucleic acids that bind to the target sample are recovered and purified. The nucleic acids binding to target peptide(s) or protein(s) may be recovered from the Western blot membrane by heating the excized protein band in water, for example at a temperature ranging from 80°C to 99°C. Preferably, the nucleic acids are recovered in a buffer. In preferred embodiments, in step c) the nucleic acids binding to target peptide(s) or protein(s) are recovered from the Western blot membrane using a buffer containing urea and sodium dodecyl sulfate. The recovering in buffer may be performed at a temperature in a range from 80°C to 99°C. Urea and sodium dodecyl sulfate have been shown to provide good recovery results. Particularly in view of generally very low amounts of nucleic acids binding to the target, a recovery of as many aptamers as possible is advantageous to provide as many copies as possible for the following amplification process. A concentration in a range of 1 M to 2 M urea and/or in a range of 0.01 M to 0.1 M of SDS may be preferred. The recovery buffer may further comprise in a range of 0.05 M to 0.1 M NaHCO₃.

The recovered nucleic acids may be purified before being used in further SELEX cycles to remove proteins and/or residual buffer components. In embodiments, in step c) the recovered nucleic acids are purified, preferably by spin column-based nucleic acid purification, phenol-chloroform extraction and/or nucleic acid precipitation. Phenol-chloroform extraction is a liquid-liquid-extraction technique. While proteins will partition into the organic phase, the nucleic acids will partition into the aqueous phase. This purification will separate the recovered binding nucleic acids from proteins that may have been released together with the nucleic acids. Spin column-based nucleic acid purification is a solid phase extraction method to purify nucleic acids from proteins by binding to the solid phase of silica. Nucleic acid precipitation using alcohol such as ethanol is a further method used to purify and/or concentrate nucleic acids by adding the alcohol as an antisolvent.

The purified nucleic acids may be washed with water or a buffer to remove contaminations from the recovery and purification process before being used for amplification. Washing of the recovered nucleic acids may already be included in the first SELEX round, or may start in the second round. Starting only in the second round may have the advantage not to reduce or remove small amounts of binding aptamers. In parallel to the enrichment of a specific aptamer during the SELEX process the washing procedure may be intensified, for example by using higher temperatures, more concentrated buffers and/or stronger ingredients.

In embodiments, in step b) in addition to the target sample a sample for negative-selection containing denatured peptides and/or proteins is provided on the Western blot. A blank sample provides the advantage to absorb unspecifically binding aptamers. Preferably, a mixture of different proteins and/or peptides may be used as a blank sample. Synthetic or natural peptides or proteins may be used, preferably a (whole) cell lysate or a protein fraction thereof.

In the first step of a SELEX or a click SELEX procedure a candidate mixture is provided. Generally, the starting candidate mixture of nucleic acids may be a DNA library. Suitable DNA libraries are commercially available. Such libraries may contain about 10¹⁵ single strand sequences. In the first cycle in a range of 500 pmole to 1 nmole of the DNA library may be used to be incubated with the target sample, preferably for example on a Western blot. Incubation time may be in range of 1 hour to 3 hours. After incubation, the unbound sequences will be removed and the binding aptamers recovered as described above. The amplification preferably is performed by PCR. After the amplification one strand of the double-stranded nucleic acid as amplified in step (d) is removed. The method of displacing one strand of the nucleic acid can be made according to principles and properties known to those of ordinary skill in the art. Single-stranded DNA can be achieved by selective lambda exonuclease digestion of one strand. The thereby received aptamers may then again be subjected to one or more in vitro selection cycles until an enrichment of target-binding sequences can be detected. The steps a) to e) may be repeated several times, for example for five to 15 cycles. Advantageously, it was seen that already in five to ten SELEX rounds, sequences that strongly bind to the selected protein target were successfully enriched.

In the second and following SELEX cycles the amount of the DNA to be incubated with the target sample, preferably on a Western blot, may be reduced. In the second cycle the amount of the DNA may be in a range of 15 pmole to 60 pmole. In the following cycles the amount of the DNA may be in a range of 2 pmole to 20 pmole. In the second and following SELEX cycles also the contact period of target sample and DNA may be reduced, for example from 3 hours in the second cycle to 2 hours in the following cyles to 1 hour in the fifth and following cycles.

The copper-catalyzed alkyne-azide cycloaddition (CuAAC or click chemistry) may advantageously be used to modify the candidate mixture of nucleic acids. In embodiments, in step a) the candidate mixture of nucleic acids comprises at least one nucleotide that is modified to comprise a functionalization introduced by click chemistry. The DNA library used for the selection for example may instead of thymidine contain ethynyldeoxyuridine (EdU), which can be modified by a click reaction with various azides. The 1,3 dipolar cycloaddition of an azide to an alkyne has been proven useful for click chemistry. In embodiments, the modified nucleotide comprises an alkyne-modified nucleobase that is further modified via a 1,3 dipolar cycloaddition of an azide to yield an azide-alkyne modified nucleobase. The modification is introduced via the azide. Suitable azides are commercially available or can be synthesized. The azide preferably comprises a group selected from benzyl and indole. Benzyl and indole modified libraries advantageously may improve the binding properties of the nucleic acids to the protein target.

The modification advantageously increases the chemical variability of the DNA library. As such, the four DNA bases have limited possibilities of chemical interaction with other molecules, e.g. peptides and proteins. By modification with other chemical groups, e.g. benzyl or indole, an increase in possible interactions with the target proteins beyond the nucleid acid molecules as such is provided, which advantageously may increase the chances of success of the selection. Benzyl-azide for example introduces the benzyl group into the alkyne-modified DNA library, which resembles the phenylalanine sidechain. Advantageously, groups such as benzyl and indole do not prevent the PCR amplification of the nucleic acids. After the lambda exonuclease digestion, the modification is reintroduced by CuAAC, and the thereby-obtained library is used for the next SELEX cycle.

In embodiments using click SELEX, equally azide-modified competitor oligonucleotides may be added to the library during the incubation with the target sample. A further preferred competitor are polyanions such as dextran sulfate. A use of azide-modified competitor oligonucleotides or polyanions provides the advantage of reducing unspecific binding of the click-modified DNA to other proteins. Due to a click functionalization with the same azide as the library, this competitor oligonucleotide has similar interaction capabilities as the modified DNA library. In these embodiments, the interaction of binding sequences with the target peptide or protein may be based on a combination of the sequence and the modification.

Another aspect of the invention relates to the use of the method to identify or produce an aptamer for the identification of a denatured peptide or protein. By performing the SELEX or click-Selex method using a target sample providing one or more peptides and/or proteins in a denatured form, preferably on a Western blot, functional aptamers are generated which advantageously lead to reproducible detection results in the Western blot. Preferably, the aptamer is usable in a Western blot assay.

The method may be used, for example, for the identification of cancer specific proteins, so-called "cancer cell markers". As a target sample cancer tissue or cancer cell lines, such as the prostate cancer cell lines PC3 and LNCaP cells, may be used. The cells may be split into membrane and cytosolic fractions and the protein fractions separated by SDS-PAGE. Subsequently, the proteins may be transferred to a nitrocellulose membrane by the Western blot method, and the membrane used as matrix for the selection of aptamers specific for proteins of cancer cells, as described above.

A further aspect of the invention relates to an aptamer, wherein the aptamer has a nucleotide sequence selected from the group comprising 5'-AACAAGANANACACGANGGGGGCACACCAAAGCACCGNNCGAAA-3' (SEQ ID NO: 1) and 5'-NNAACACCCACNCACGCCAANCCCACCACCCCNACACNCCCAC-3' (SEQ ID NO: 2), wherein N is selected from T or ethynyl-dU.

Preferably, N is ethynyl-dU. Ethynyl-dU may be coupled via a 1,3 dipolar cycloaddition to an azide comprising a group selected from benzyl and indole. The aptamer of SEQ ID NO: 1 provides a specific and affine sequence against the recombinant protein glutathione S-transferase-tagged human p53 tumor suppressor protein (GST-p53). It could be shown that up to 10 ng of GST-p53 could be detected and fluorescently visualized. A comparison with common p53 antibodies showed an improved specificity of the aptamer. In embodiments of the aptamer of SEQ ID NO: 1, wherein a benzyl modification is used in the method, the binding of the thus selected aptamer may depend on the benzyl modification and without this, no signal will be visible.

The aptamers selected by the method of the invention, particularly the sequences of the aptamers of SEQ ID NO: 1 and 2 may be labelled with a biotin label and/or with a fluorophore. This provides the possibility to visualize the aptamers in different ways without affecting the binding to the target protein.

Unless defined otherwise, the technical and scientific terms used herein have the same meaning as commonly understood by one of ordinary skill in the art to which the invention belongs.

The Figures and Examples which follow serve to illustrate the invention in more detail but do not constitute a limitation thereof.

The figures show:
- Figure 1: the results of a fluorescent binding test of the benzyl-functionalized monoclone of SEQ ID NO: 1 selected according to one embodiment of the method, shown on the right, and the starting library ("Bibliothek") on the left.
- Figure 2: the results of binding studies of the 7^{th} and 10^{th} SELEX round visualized with IR-fluorescence according to a second embodiment of the method of the invention.
- Figure 3: the results of a fluorescent binding test of the indol-functionalized monoclone of SEQ ID NO: 2 selected according to the second embodiment of the method, in PC3 cells and THP-1 cell line.

### Materials:

### Nucleic Acids:

| name | specification | supplier |
|---|---|---|
| Library (OW1_Cy5): 5'-CY5-AGC CAC GGA AGA ACC AGA NNN NNN NNN NNN NNN NNN NNN NNN NNN NNN NNN NNN NNN NNN NNG CAG AAG CGA CAG CAA CA-3'(SEQ ID NO: 3) | Library | Ella Biotech GmbH, Germany |
| N: dA, dC, dG, Ethynyl-dU (EdU) (1:1:1:1) (DNA) | | |
| Panacea: 5'-NNN NNN NNN NNN NNN NNN NNN NNN NNN NNN NNN NNN NNN A-3 '(SEQ ID NO: 4) | Click -competitor | Ella Biotech GmbH, Germany |
| N: dA, dC, dG, Ethynyl-dU (EdU) (1:1:1:1) (DNA) | | |
| 5'-Biotin-X*AG CCA CGG AAG AAC CAG A -3' (SEQ ID NO: 5) | forward primer (OW1 fw bio) | Ella Biotech GmbH, Germany |
| *X=C18 Spacer | | |
| 5'-IR-800CW- X*AG CCA CGG AAG AAC CAG A-3' (SEQ ID NO: 5) | forward primer (OW1 fw IR-800) | Ella Biotech GmbH, Germany |
| Phosphate-TGT TGC TGT CGC TTC TGC (SEQ ID NO: 6) | reverse primer (OW1 rev P) | Ella Biotech GmbH, Germany |

### Proteins:

| name | specification | supplier | Catalogue no. |
|---|---|---|---|
| *Pwo* DNA polymerase | 2.5 U / µl | Genaxxon, Germany | M3002.0100 |
| λ-Exonuclease | 10 U / µl | Thermo Scientific | EN0562 |
| Recombinant human GST-p53 | | Abcam, UK | Ab43615 |
| Bovine Serum Albumine (BSA) | | AppliChem, Germany | 232-963-2 |
| Streptavidin-Horseradish peroxidase | | GE-Healthcare Amersham, UK | RPN-1231-2ML |

### Cell lines:

| name | supplier | Catalogue no. |
|---|---|---|
| PC3-LN | ProQinase, Germany | |
| LNCaP | DSMZ, Germany | ACC 256 |

### Chemicals:

| name | supplier | Catalogue no. |
|---|---|---|
| Tris(4-(3-hydroxy-propoyl)-[1,2,3]triazol-1-ylmethyl)amine (THPTA) | Baseclick, Germany | BCMI-006-5 |
| Copper sulphate | Sigma-Aldrich | 451657-10G |
| 5-Ethynyl-5'-*O*-triphosphate-2'-deoxyuridine (Ethinyl-dUTP) | Baseclick, Germany | BCT-08 |
| dATP | Genaxxon bioscience, Germany | M3018.0020 |
| dCTP | Genaxxon bioscience, Germany | M3019.0020 |
| dGTP | Genaxxon bioscience, Germany | M3020.0020 |
| TWEEN® 20 | Merck Millipore, Germany | 8170721000 |
| TRIS | Carl ROTH, Germany | 201-064-4 |
| Glycin | Carl ROTH, Germany | 200-272-2 |
| NaCl | Labochem international, Germany | LC-5932.1 |
| MgCl₂ | Carl ROTH, Germany | 2189.2 |
| CaCl₂ | Carl ROTH, Germany | 5239.1 |
| NaHCO₃ | Carl ROTH, Germany | 8551.1 |
| UREA | Carl ROTH, Germany | 3941.1 |
| EDTA | AppliChem, Germany | 200-449-4 |
| SDS | Carl ROTH, Germany | 2326.2 |
| Dextrane sulfate | Sigma-Aldrich | 9011-18-1 |
| Agarose | Carl ROTH, Germany | K297.3 |

### Miscellaneous:

| name | supplier | Catalogue no. |
|---|---|---|
| Amicon 10 K MWCO Columns | Merck Millipore, Germany | UFC501096 |
| NucleoSpin® Gel and PCR Clean-up | Macherey-Nagel, Germany | 740609.250 |
| ECL-substrates A and B | Thermo Scientific Pierce, USA | 32106 |
| TOPO TA cloning kit | Thermo Fisher Scientific, USA | K4575J10 |
| NucleoSpin Plasmid kit | Macherey-Nagel, Germany | 740588.250 |
| NucleoSpin® Gel and PCR Clean-up | Macherey-Nagel, Germany | 740609.250 |
| BCA Protein Assay Kit | Thermo Scientific Pierce, USA | 23225 |
| Pierce® ECL Western Blotting Substrate | Thermo Fisher Scientific, USA | K4575J10 |

### Buffers example 1:

| name | specification |
|---|---|
| TBST | 20 mM TRIS, 137 mM NaCl, 0.1 % TWEEN® 20, pH 7.6 |
| TBST-MC | 20 mM TRIS, 137 mM NaCl, 1 mM MgCl₂, 1 mM CaCl₂, 0.1 % TWEEN® 20, pH 7.6 |
| Recovery Buffer | 50 mM NaHCO₃, 1,25 M UREA, 6,25 mM EDTA, 0.5 % SDS |
| WetBlot Buffer | 25 mM TRIS, 192 mM Glycine |
| SDS-PAGE running Buffer | 25 mM TRIS, 192 mM Glycine, 3.4 mM SDS |

### Buffers example 2:

| name | specification |
|---|---|
| Homogenizing buffer | 5 mM Tris, 300 mM Sucrose, 0.1 M EDTA, 1 mM PMSF, pH 7.4 |
| 4x Laemmli buffer | 78.1 mM Tris, 12.5 % Glycerol, 6.3 % 2-Mercaptoethanol, 0.003 % Bromophenol Blue, 1 % SDS, pH 6.8 |
| TBS-T | 20 mM Tris, 137 mM NaCl, 1 mM MgCl₂, 1 mM CaCl₂, 0.1 % TWEEN® 20, pH 7.6 |
| Recovery Buffer | 0.1 M NaHCO₃, 1 % SDS, pH 8.0 |
| Transferring Buffer | 2.5 mM Tris, 2 % Glycine |
| SDS-PAGE Running Buffer | 25 mM Tris, 200 mM Glycine, 0.1 % SDS |

### Example 1

The method targeting GST-p53 and employing a Benzyl-modification

### 1.1 - Preparing a candidate mixture of nucleic acids

The single stranded DNA starting library comprising 44 wobble positions (OWl_Cy5: 5'-CY5-AGC CAC GGA AGA ACC AGA NNN NNN NNN NNN NNN NNN NNN NNN NNN NNN NNN NNN NNN NNN NNG CAG AAG CGA CAG CAA CA-3'; N: dA, dC, dG, Ethynyl-dU (1:1:1:1)) was synthesized and HPLC purified Ella Biotech, Martinsried, Germany.

The library was further functionalized with the Benzyl-azide in a Cu(I) catalysed azide-alkyne cycloaddition (CuAAC reaction) as follows: 5 µl of a 100 µM DNA solution were mixed with 65 µl H₂O and 10 µL 10 x Phosphate Buffer, pH 7.0. Then 10 µl 10 mM azide solution were added and the reaction was started by addition of 10 µl freshly prepared catalyst solution (4 mM tris(3-hydroxypropyltriazolylmethyl)amine (THPTA), 1 mM CuSO₄, 25 mM Sodium ascorbate, incubated for 10 min at room temperature before addition to the DNA mixture). The reaction mixture was incubated for 1 h at 37 °C, 350 rpm and the functionalized ssDNA was purified with an Amicon 10 K MWCO column according to the manufacturers recommendation. Washing was performed once with 400 µL H₂O. The DNA candidate mixture was heated up to 95 °C for 5 min and let cool down to room temperature.

### 1.2 - Preparation of the target sample

1 µg recombinant human GST-p53 expressed and purified by abcam, UK and 20 µg of HEK (Human Embryonic Kidney) cell lysate for negative-SELEX as well as a molecular weight marker were loaded onto a 12.5 % TRIS-Glycin SDS-PAGE gel and gelelectrophoresis was performed for 15 min at 100 V followed by 60 min at 170 V. A Western blot using a nitrocellulose membrane was prepared using the Mini-Trans-Blot cell (BioRad) according to the manufacturers recommendation. Afterwards the membrane was blocked with 5 % BSA in TBST for 1 h at room temperature on a shaking platform. The membrane was washed 3 times for 5 min with TBST while shaking and the piece containing endogenous p53 was cut out from the HEK lysate lane.

### 1.3 - Contacting the candidate mixture with the target sample and partitioning the nucleic acids that bind to GST-p53 from those that do not bind

All of the DNA candidate mixture was added to 5 mL of TBST-MC and incubated with the membrane for 3 h at room temperature while shaking. The membrane was washed 3 times for 5 min with TBST-MC while shaking before the piece containing GST-p53 was cut out and added to a tube containing 200 µL recovery buffer. The sample was heated to 99 °C for 10 min while shaking at 850 rpm. Purification was performed with an Amicon 10 K MWCO column according to the manufacturers recommendation. Washing was performed 3 times with 400 µL H₂O and the last centrifugation step was prolonged to 10 min to reduce the final volume. The resulting sample was used for the subsequent PCR Amplification.

### 1.4 - Amplification of the binding nucleic acids

The purified DNA (enriched library) was used in a 100 µL PCR reaction that was prepared on ice according to the scheme displayed in table 1. 0.1 pmol of OW1-Cy5 was used as positive control and H₂O without DNA as negative control. The samples were cycled in a Mastercycler® nexus (Eppendorf) for 10 cycles.

**Table 1: PCR scheme**

| Reagent | Stock-Conc. | Final-Conc. |
|---|---|---|
| Water | | |
| DNA solution | | |
| PWO-Buffer | 10X | 1X |
| dN*TPs | 25 mM each | 250 µM each |
| F-Primer (biotinylated) | 100 µM | µM |
| R-Primer (-P) | 100 µM | 1 µM |
| PWO-Pol. | 2.5 U/µl | 2.5 U |

| | | |
|---|---|---|
| dN*TPs: dATP / dCTP / dGTP / Ethynyl-dUTP | | |

The cycling scheme was as follows: 2 min at 95°C, followed by 30 sec at 95 °C / 30 sec at 62 °C / 1 min at 72 °C for X cycles, 2 min at 72 °C, storage at 10 °C. After 10 cycles the PCR reaction was split into 10 PCR tubes (10 µL each), 10 µL of the controls were also transferred into fresh PCR tubes. 12 fresh 100 µL PCR reactions were prepared with these on ice according to the scheme in table 1. The samples were cycled until the desired product band could be detected (after another 20 cycles) on an ethidium bromide stained 4 % agarose gel. The dsDNA PCR products were purified with the NucleoSpin® Gel and PCR Clean-up kit according to the manufacturers recommendation. 2 PCR samples were pooled on one column and the DNA was eluted 2 times 5 min with 25 µL H₂O at 65 °C. The purified samples were pooled (250 µL total) and a backup of this SELEX round (10 % of the purified dsDNA) was stored at -20 °C.

### 1.5 - Single strand displacement

Purified dsDNA PCR product was supplemented with 25 µl 10 X exonuclease reaction buffer and 10 µl lambda exonuclease were added. The mixture was incubated for 60 min at 37 °C, 350 rpm and checked on an ethidium bromide stained 4 % agarose gel for remaining dsDNA. If there was no dsDNA left, the ssDNA digestion product was purified with the NucleoSpin® Gel and PCR Clean-up kit according to the manufacturers recommendation. The DNA was eluted 2 times 5 min with 25 µL H₂O at 65 °C.

### 1.6 - Functionalization of the enriched library

For the next SELEX round the enriched single stranded library was again further functionalized with the Benzyl-azide in a CuAAC reaction as follows: 50 µl of the ssDNA solution were mixed with 20 µl H₂O and 10 µL 10 x Phosphate Buffer, pH 7.0. Then 10 µl 10mM azide solution were added and the reaction was started by addition of 10 µl freshly prepared catalyst solution (4 mM THPTA, 1 mM CuSO₄, 25 mM Sodium ascorbate, incubated for 10 min at room temperature before addition to the DNA mixture). The reaction mixture was incubated for 1 h at 37 °C, 350 rpm and the functionalized ssDNA was purified with an Amicon 10 K MWCO column according to the manufacturers recommendation. Washing was performed once with 400 µL H₂O. The DNA candidate mixture was heated up to 95 °C for 5 min and let cool down to room temperature. The ssDNA concentration was determined (see table 2) via A₂₆₀ on a Nanodrop (Thermo Fisher) and all of the ssDNA sample was afterwards used in the next SELEX round.

### 1.7 - 2^{nd} to 5^{th} SELEX round

The next SELEX round was performed as described for the first round (chapters 1.2 - 1.6). In total 5 rounds of SELEX were performed. To increase the selection pressure the washing times were increased, the binding time was reduced and a click competitor (Panacea) functionalized with the Benzyl-azide in the same way as the candidate mixture was added as summarized in the following table 2:

**Table 2: Summary of SELEX strategy**

| SELEX round | Amount of funcitionalized ssDNA (pmol) | Binding time (h) | Washing with TBST-MC | Amount of click competitor (pmol) | # of PCR cycles needed for amplification |
|---|---|---|---|---|---|
| 1 | 500 | 3 | 3 x 5 min | - | 10 + 20 |
| 2 | 16.8 | 3 | 3 x 5 min | - | 10 + 20 |
| 3 | 4.2 | 3 | 3 x 5 min | - | 10 + 15 |
| 4 | 2.6 | 3 | 3 x 10 min | 500 | 10 + 15 |
| 5 | 2.4 | 1 | 6 x 10 min | 500 | 10 + 10 |

### 1.8 - Amplification of enriched library and OW1-Cy5

The enriched library after round 5 as well as the OW1-Cy5 library were amplified in ten 100 µL PCR reactions each as described in chapter 1.4. 1 µL of the sample resulting from the 5^{th} SELEX round was used per PCR reaction and 0.1 pmol of OW1-Cy5, respectively. After 15 PCR cycles the dsDNA PCR products were visualized on an ethidium bromide stained 4 % agarose gel and afterwards purified with the NucleoSpin® Gel and PCR Clean-up kit according to the manufacturers recommendation. The DNA was eluted 2 times 5 min with 25 µL H₂O at 65 °C.

### 1.9 - Chemiluminescent binding assay

Single strand displacement and functionalization were performed as described in chapters 1.5 and 1.6 and three GST-p53 Western blots were prepared as described in chapter 1.2 except that the piece containing endogenous p53 was not cut out from the HEK lysate lane this time. 10 pmol of functionalized and non-functionalized enriched library as well as functionalized OW1, respectively, were added to 3 x 5 mL TBST-MC containing 0.1 mg/mL Dextrane sulphate and 500 pmole functionalized Panacea click competitor, each. The mixtures were added to one Western blot each and incubated on a shaking platform for 1 h at room temperature. The membranes were washed 3 times for 5 min with TBST-MC while shaking before 5 mL per blot of 1:1000 Streptavidin-HRP in TBST-MC were added. After 30 min of incubation at room temperature on the shaker the membranes were washed again for 3 x 2 min, 1 x 5 min, 1 x 10 min and 1 x 15 min. 7.5 mL of ECL-substrate A and 7.5 mL of ECL-substrate B were mixed and 5 mL of the mixture were added per Western blot. After one minute of incubation the ECL-substrate mixture was exchanged for 5 mL TBST-MC and the chemiluminescence was detected on a VersaDoc (BioRad).

After 5 SELEX rounds the binding of the starting library OW1 was compared to the binding of the benzyl-functionalized and non-functionalized enriched library and a stronger binding of the functionalized enriched library was seen. This result demonstrates that nucleic acids that bind to the GST-p53 target were successfully enriched. Since a benzyl-modification was used for the selection, without the benzyl-modification no binding was detected.

### 1.10 - Cloning and sequencing of the enriched SELEX pool

Before cloning the sequences into a bacterial vector, a poly-A-overhang needs to be added by Taq-PCR.
Sample: 1 µl enriched SELEX pool + 19 µl ddH₂O
PTC: 1 µl library + 19 µl ddH₂O
NTC: 20 µl ddH₂O

**Table 3: Buffer conditions Taq PCR:**

| Reagent | Stock-Conc. | Final-Conc. |
|---|---|---|
| Water | | |
| DNA solution | | |
| Taq-Buffer | 10X | 1X |
| dN*TPs | 25 mM each | 250 µM each |
| F-Primer (biotinylated) | 100 µM | 1 µM |
| R-Primer (-P) | 100 µM | 1 µM |
| Taq-Pol. | 2.5 U/µl | 2.5 U |

The temperature steps were the same as for the Pwo-PCR described before. 10 cycles were done.

Cloning was performed according to standard protocol provided by the manufacturer with the TOPO TA cloning kit (Thermo Fisher).

Transformation: One aliquot TOP10 *E*.*coli* cells (provided by Thermo Fisher) was thawn on ice. To 25 µl bacteria suspension, 2 µl of cloning reaction were added and incubated for 10 min on ice. A heat shock was induced at 42 °C for 30 sec. Cells were put on ice for 5 min before 250 µl LB medium was added and the bacteria were incubated at 37 °C for 1 h, shaking at 300 rpm. Then, they were spread on agar plates containing ampicillin as a selection antibiotic and grown over night at 37 °C. On the next day, small amounts of the grown colonies were used as samples for a Taq PCR to control for successful cloning and transformation. Sample preparation: A small fraction was taken from each colony, resuspended in 5 µl ddH₂O, incubated at 95 °C for 10 min and centrifuged for 10 min at 21130 rcf. The supernatant was used as PCR template. In the cases in which the colony PCR lead to an amplification product of the correct length, small amounts of the colonies were taken and incubated overnight at 37 °C shaking at 120 rpm in 5 ml LB-medium containing 100 µg/ml ampicillin each. On the next day, the plasmids were purified according to standard protocol of the NucleoSpin Plasmid kit (Macherey Nagel) and sent to GATC for Sanger sequencing.

### 1.11 - Fluorescent binding assay of monoclone/single sequence

An IRD800CW-labelled forward-Primer was used for amplification of the DNA as described in chapter 1.8 and 1.4, respectively. Afterwards single strand displacement and functionalization was performed as described in chapters 1.5 and 1.6 and three GST-p53 Western blots were prepared as described in chapter 1.2 except that the piece containing endogenous p53 was not cut out from the HEK lysate lane this time. 10 pmole of functionalized and non-functionalized enriched library as well as functionalized OW1, respectively, can be added to 3 x 5 mL TBST-MC containing 0.1 mg/mL Dextrane sulphate and 500 pmole functionalized Panacea click competitor, each. The mixtures were then added to one Western blot each and incubated on a shaking platform for 1 h at room temperature. The membranes were washed 3 times for 5 min with TBST-MC while shaking before the fluorescence was visualized using an Odyssey Scanner (Li-Cor).

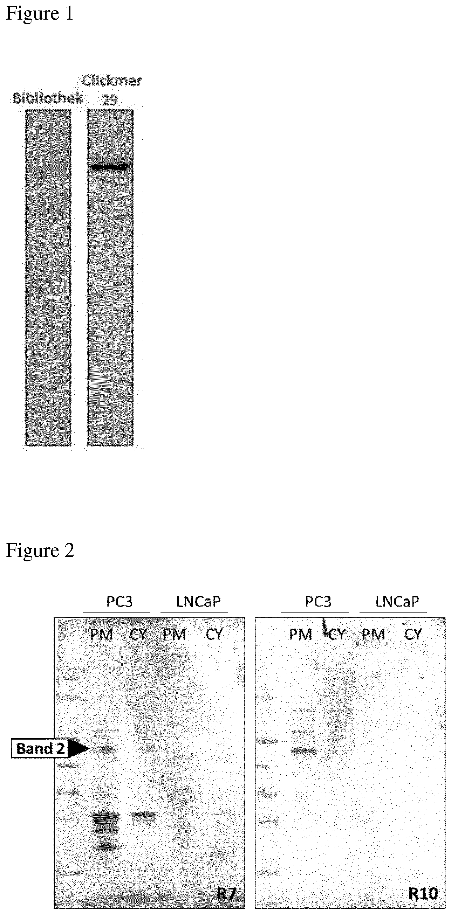

The Figure 1 shows the results of the fluorescent binding test of the monoclone of the aptamer of SEQ ID NO: 1. The benzyl-functionalized Sequence (SEQ ID NO: 1), shown on the right, was labeled with the fluorescent dye IRD800CW and showed a stronger binding to GST-P53 than the starting library ("Bibliothek"), shown on the left, as can be taken from the Figure 1.

### Example 2

The method targeting membrane proteins of PC3 cells

### 2.1 - Preparing a candidate mixture of nucleic acids

The single stranded DNA starting library comprising 44 wobble positions (OW1: 5'- AGC CAC GGA AGA ACC AGA -N44- GCA GAA GCG ACA GCA ACA -3'; N: dA, dC, dG, Ethynyl-dU (1:1:1:1)) was synthesized and HPLC purified Ella Biotech, Martinsried, Germany.

The library was further functionalized with the Indole-azide in a Cu(I) catalysed azide-alkyne cycloaddition (CuAAC reaction) as follows: 5 µl of a 100 µM DNA solution were mixed with 65 µl H₂O and 10 µL 10 x Phosphate Buffer, pH 7.0. Then 10 µl 10 mM azide solution were added and the reaction was started by addition of 10 µl freshly prepared catalyst solution (4 mM THPTA, 1 mM CuSO₄, 25 mM Sodium ascorbate, incubated for 10 min at room temperature before addition to the DNA mixture). The reaction mixture was incubated for 1 h at 37 °C, 650 rpm and the functionalized ssDNA was purified with a NucleoSpin® Gel and PCR Clean-up column according to the manufacturers recommendation. Elution was performed four times with 15 µL ddH₂O. The DNA candidate mixture was heated up to 95°C for 3 min and let cool down to room temperature.

### 2.2 - Preparation of the target sample

PC3 and LNCaP cells were fractionated to the membrane and cytosolic fraction. At least 2 million cells were used for the subcellular fractionation, cultured in three to four T75 flasks. First, the media was discarded and cells washed with cold DPBS. Subsequently, the cells were detached from the surface by adding cold DPBS, pipetting and scraping. Detached cells in DPBS were spun down at 1000 x g and RT for 3 min. The supernatant was discarded and pellet was resuspended in 750 µl ice cold and freshly prepared homogenizing buffer containing 0.3 M sucrose, 5 mM Tris-HCl, 0,1 mM EDTA, 1 mM PMSF, pH 7.4. Cells were homogenized on ice, applying 100 strokes with the homogenizing potter. Then, the solution was transferred to a 2-mL vial and centrifuged two times at 800 x g for 5 min at 4°C. Pellet, containing intact cells and nuclei, was discarded and the supernatant was centrifuged at 20,000 x g for 2 hours at 4°C, yielding cytosolic fraction in the supernatant and enriched membrane fraction in the pellet. To dilute cytosolic proteins in the pellet as much as possible, the pellet was washed with cold DPBS, resuspended in 500 µl homogenizing buffer and centrifuged at 20,000 x g for 1 hour at 4°C, again. The last centrifugation step was repeated and, finally, the pellet was resuspended in 250 µl 5 mM Tris and 1 mM PMSF, pH 7.4.

Concentration of the protein fractions was determined with Pierce® BCA Protein Assay Kit according to the manufacturers recommendation.

1 µg of each protein fraction (PC3.PM as a target, and PC3.CY, LNCaP.PM, LNCaP.CY for negative-SELEX) was mixed with 4x Laemmli buffer and heated up at 95°C for 5 minutes. Denaturized protein samples and additional PageRuler Prestain Protein Ladder were loaded onto a 10 % Tris-Glycin SDS-PAGE gel and electrophoresis was performed at 50 V for 5 min followed at 150 V until the lowest protein ladder band came to the end of the chamber. Semi-dry transfer of proteins was performed with the Semi-dry transfer cell (BioRad) to the nitrocellulose membrane. Afterwards, the membrane was blocked in 5 % BSA in TBS-T buffer for 1 hour at RT on a rotator. Finally, the membrane was washed three times with TBS-T for 5 min on a rotator.

### 2.3 - Contacting the candidate mixture with the target sample and partitioning the nucleic acids that bind to membrane proteins of PC3 cells (PC3.PM) from those that do not bind

1 nmole of the DNA candidate mixture was added to 2.5 mL of TBS-T with 1 nmole of indole-modified click-competitor and 0.01 mg/ml of dextran sulphate and incubated with the membrane for 3 h at RT on a rotator. The membrane was washed 3 times for 5 min with TBS-T while rotating before the lane with membrane proteins of PC3 cells was cut out and added to a tube contacting 1 mL of recovery solution. The sample was incubated at 99°C for 10 min and 1,400 rpm. The sample was purified with EtOH precipitation and the pellet resuspended in 80 µl of ddH₂O. The resulting sample was used for the subsequent PCR Amplification.

### 2.4 - Amplification of the binding nucleic acids

Recovered and purified sequences were amplified before going to the next round. PCR reaction volume was 800 µl. Additionally, a no template control (only ddH₂O instead of the sample) and a positive control (OW1 library) were amplified with the PCR reaction. The PCR master mix was prepared on ice using freshly prepared ddH₂O. During the selection, thymidine in the dNTPs mixture was replaced with EdU and instead of usual primers, a biotinylated forward and phosphorylated reverse primers were used. 100 µl of the reaction mixture were aliquoted into 0.5 ml vials and transferred from ice directly to a preheated PCR cycler.

**Table 4: PCR scheme.**

| Reagent | Stock C | Final C | 1x |
|---|---|---|---|
| 10x PWO buffer | 10x | 1x | 10 µl |
| fwd primer (bio) | 100 nM | 1 µM | 1 µl |
| rev primer (pho) | 100 nM | 1 µM | 1 µl |
| dNTPs (each) | 25 mM | 1.25 µM | 1 µl |
| PWO Polymerase | 2.5 U/µl | 0.025 U/µl | 1 µl |
| ddH₂O | | | 76 µl |
| Sample | | | 10 µl |

**Table 5: Settings used in the PCR reaction.**

| Step no | Step | Temperature | Time |
|---|---|---|---|
| 1 | Initial denaturation | 95°C | 2 min |
| 2 | Denaturation | 95°C | 30 s |
| 3 | Annealing | 62°C | 30 s |
| 4 | Elongation | 72°C | 1 min |
| 5 | Repetition of step 2-4 | | |
| 6 | Final elongation | 72°C | 2 min |
| 7 | Cooling down | 4°C | ∞ |

The samples were cycled until the desired product band could be detected on an ethidium bromide stained 4 % agarose gel. The dsDNA PCR products were purified with the NucleoSpin® Gel and PCR Clean-up kit according to the manufacturers recommendation. The DNA was eluted 4 times with 25 µl of ddH₂O.

### 2.5 - Single strand displacement

After purification, 10 µL of Lambda Exonuclease buffer and 1 µL of Lambda Exonuclease were added to 90 µL dsDNA. The mixture was incubated at 37°C for 15 min and 1,000 x g in a thermomixer. The completeness of digestion was checked with the agarose gel electrophoresis, where the band at 80 bp should not be visible anymore, but only the band of ssDNA at 50 bp. After digesting one strand of DNA, the solution was purified with the NucleoSpin® Gel and PCR Clean-up kit according to the manufacturers recommendation. The DNA was eluted 4 times with 15 µl of ddH₂O.

### 2.6 - Functionalization of the enriched library

For the next SELEX round all of the enriched single stranded library was again further functionalized with the Indole-azide in a CuAAC reaction as follows: 60 µl of the ssDNA solution were mixed with 10 µl ddH₂O and 10 µL 10 x Phosphate Buffer, pH 7.0. Then, 10 µl 10 mM azide solution were added and the reaction was started by addition of 10 µl freshly prepared catalyst solution (4 mM THPTA, 1 mM CuSO₄, 25 mM Sodium ascorbate, incubated for 10 min at room temperature before addition to the DNA mixture). The reaction mixture was incubated for 1 h at 37 °C, 650 rpm and the functionalized ssDNA was purified with a NucleoSpin® Gel and PCR Clean-up kit according to the manufacturers recommendation. Elution was performed 4 times with 25 µL ddH₂O. The DNA candidate mixture was heated up to 95 °C for 3 min and let cool down to room temperature. The ssDNA concentration was determined via A₂₆₀ on a Nanodrop (Thermo Fisher) and all of the ssDNA sample was afterwards used in the next SELEX round.

### 2.7 - 2^{nd} to 7^{th} SELEX round

The next SELEX round was performed as described for the first round (chapters 2.2 - 2.6). In total 7 rounds of SELEX were performed. To increase the selection pressure the washing times were increased, the binding time was reduced and the amount of negative selection target (LNCaP.PM) was increased.

**Table 6: Summary of SELEX strategy.**

| SELEX round | Target | | Incubation | | | Washing | PCR | |
|---|---|---|---|---|---|---|---|---|
| | Selection | µg | pmole (OW1) | Competitors | Time | Time & Volume | Number of cycles | volume |
| 1 | POSITIVE: PC3.PM NEGATIV E: PC3.CY LNCaP.PM | PC3.PM: 1 PC3.CY: 1 LNCaP.C Y: 1 | 1,000 | 1:1 click-compet itor & 0.01 mg/ml dextran sulphate | 3 h | 3x 5 min 3 ml | 18 | 800 µl |
| 2 | | | 15 | | | | 16 | 800 µl |
| 3 | | | 14 | | 2 h | 3x 10 min 5 ml | 16 | 800 µl |
| 4 | +NEGATI VE: LNCaP.PM | +LNCaP. PM: 2 | 28 | | | | 16 | 800 µl |
| 5 | | | 24 | | 1h | 3x 15 min 7 ml | 16 | 800 µl |
| 6 | | | 10 | | | | 18 | 1000 µl |
| 7 | | +LNCaP. PM: 10 | 10 | | | | 16 | 1000 µl |

### 2.8 - Amplification of enriched pools and initial OW1 library

Sequences, obtained after the second, fourth, sixth, and seventh round as well as the OW1 library were amplified in 800-1000 µl of PCR reaction mix, for which approximately 0.1 pmole of selected sequences per 100 µl were used. After 8 PCR cycles, the dsDNA PCR products were purified with the NucleoSpin® Gel and PCR Clean-up kit according to the manufacturers recommendation. Single strand displaced and modification of ssDNA with the Indole followed as described in chapter 2.5 and 2.6.

### 2.9 - Chemiluminescent binding assay

The membrane was prepared with 1 µg of each protein fraction as described in chapter 2.2. Each membrane was incubated in 2.5 mL of TBS-T with 10 pmol of ssDNA sequences (OW1 library, 2^{nd}, 4^{th}, 6^{th}, or 7^{th} round pool), 1:1 click-competitor and 0.01 mg/ml dextran sulphate. Subsequently, tubes with the membranes were rotated for 1 hour at RT. After incubation, the membranes were washed three times with 5 ml of TBS-T for 10 minutes. Streptavidin-HRP was incubated with the membrane and, consequently, strong interaction between biotin and streptavidin was formed. For each membrane 2 ml of 1:1000 diluted Streptavidin-HRP in TBS-T were used and incubated with the membrane for 30 min at RT while rotating. Afterwards, the membrane was washed with TBS-T 3x 1 ml (2 min), 1x 2 ml (5 min), 1x 3 ml (10 min) and 1x 4 ml (15 min). Finally, the reagent with peroxidase (1 mL) and luminol (1 mL) was added and incubated for 1 min. The chemiluminescence was detected on a VersaDoc (BioRad).

### 2.10 - 8^{th} to 10^{th} SELEX round

The last three SELEX rounds were performed as described for the first round (chapters 2.2 - 2.6) with an exception that only band 2 (Figure 2) was cut out and the recovered sequences used for the next round. The cut membrane piece was recovered in 100 µl of recovery solution for 10 min at 99°C and 1,400 rpm. The sample was purified with EtOH precipitation and the pellet resuspended in 10 µl of ddH₂O. The resulting sample was used for the subsequent PCR amplification in 100 µl of PCR reaction mix using fluorophore-labelled forward primer instead of biotinylated one. Afterwards, amplified DNA was used for further PCR in 800 µl of PCR reaction mix (1 µl of PCR product/100 µl PCR reaction mix) as described in chapter 2.4.

The Figure 2 shows the binding studies of the 7^{th} and 10^{th} SELEX round visualized with IR-fluorescence. As can be seen in the Figure 2, after the seventh round, only band 2 was cut out and recovered sequences were used for the next SELEX rounds.

Other SELEX steps were done the same as described previously. To increase the selection pressure the washing times and volume were increased and the amount of the ssDNA was decreased.

**Table 7: Summary of SELEX strategy for 8^{th} to 10^{th} SELEX round.**

| SELEX round | Target | | Incubation Washing | | | | PCR | |
|---|---|---|---|---|---|---|---|---|
| | Selection | µg | pmole (OW1) | Competitors | Time | Time & Volume | Number of cycles | volume |
| 8 | POSITIVE: 2^{nd} band-PC3.PM NEGATIV E: PC3.CY LNCaP.PM LNCaP.PM | PC3.PM: 1 PC3.CY: 1 LNCaP.C Y: 1 LNCaP.P M: 1 | 10 | 1:1 click-compet itor & 0.01 mg/ml dextran sulphate | 1 h | 3x 10 min 5 ml | 20 | 100 µl |
| 9 | | | 5 | | | | 16 | 100 µl |
| 10 | | | 5 | | 2 h | 5x 10 min 5 ml | 15 | 100 µl |

### 2.11 - IR-fluorescence binding studies

The last three rounds were performed and binding visualized using IR-800CW-labelled forward primer, as the visualization needed to be directly performed for each round. For the binding studies, 10 pmol (8^{th} round) or 5 pmol (9^{th} and 10^{th} round) of Indole-modified ssDNA was used in 2.5 mL of TBS-T with additional 1:1 click-competitor and 0.01 mg/mL of dextran sulphate. The membrane was incubated for 1 h at RT while rotating, and afterwards washed 3 times with 5 mL of TBS-T for 10 min. Finally, the bound sequences were visualized using an Odyssey Scanner (Li-Cor).

### 2.12 - Cloning and sequencing of the enriched SELEX pool

Before cloning the sequences into a bacterial vector, a poly-A-overhang needs to be added by Taq-PCR. Additional sample no-template control was prepared, where only water was used instead of the template (10^{th} round SELEX pool).

**Table 8: PCR scheme for amplification with Taq-polymerase.**

| Substance | Stock Conc. | Final Conc. | Volume |
|---|---|---|---|
| Taq-Buffer | 5x | 1x | 20 µL |
| MgCl₂ | 25 mM | 2 mM (+1.5 mM) | 8 µl |
| dNTPs | 25 mM | 250 µM | 1 µL |
| fw-Primer | 100 µM | 0.5 µM | 1 µL |
| rv-Primer (without 5'-P!) | 100 µM | 0.5 µM | 1 µL |
| Taq-Polymerase | 5 U/µL | 0.025 U/µL | 1 µL |
| ddH₂O | | | 67 µL |
| Template / water | | | 1 µl |

**Table 9: Settings used in the PCR reaction with the longer final elongation time.**

| Step no | Step | Temperature | Time |
|---|---|---|---|
| 1 | Initial denaturation | 95°C | 2 min |
| 2 | Denaturation | 95°C | 30 s |
| 3 | Annealing | 62°C | 30 s |
| 4 | Elongation | 72°C | 1 min |
| 5 | Repetition of step 2-4 | | |
| 6 | Final elongation | 72°C | 5 min |
| 7 | Cooling down | 4°C | ∞ |

In total, 11 PCR cycles were performed.
Cloning was performed according to standard protocol with the TOPO TA cloning kit (Thermo Fisher).

Transformation: One aliquot TOP10 *E*.*coli* cells (provided by Thermo Fisher) was thawn on ice. To 25 µl bacteria suspension, 2 µl of cloning reaction were added and incubated for 10 min on ice. A heat shock was induced at 42°C for 30 sec. Cells were put on ice for 5 min before 250 µl LB medium was added and the bacteria were incubated at 37 °C for 1 h, shaking at 300 rpm. Then, they were spread on agar plates containing ampicillin as a selection antibiotic and grown over night at 37 °C.

On the next day, 50 single clones were picked with a tip and resuspended in 5 mL of LB-Amp-Medium (50 µg / ml Amp) for each sample. The samples were incubated over night at 37°C and 120 rpm. On the next day, 2 mL *of E*. *coli* LB-Medium were purified according to standard protocol of the NucleoSpin Plasmid kit (Macherey Nagel). After purification, 1 µl of each sample was taken and used for the PCR. The samples for which the PCR lead to an amplification product of the correct length were sent to GATC for Sanger sequencing.

### 2.13 - IR-fluorescence binding studies of monoclone/single sequence

The most enriched sequences obtained by Sanger sequencing were used for the binding assay. Clones prepared in chapter 2.12 were amplified in 3 rounds, each time taking 1 µl of the template for 100 µl PCR reaction (see Table 1). Instead of using biotinylated forward primer, an IRD800CW-labelled forward-primer was used. The amplification was done after 8 cycles. In the third round of amplification, no clones were seen on the agarose gel and the PCR was performed in 800 µl to have a sufficient amount of DNA for further binding studies. After the purification with NucleoSpin® Gel and PCR Clean-up kit, single strand displacement and functionalization was performed as described in chapters 2.5 and 2.6. The binding was tested on the membrane with 1 µg of all four proteins fractions, prepared as described in chapter 2.2. 0.5 pmol of Indole-modified ssDNA of each clone was used during 1-hour incubation with the membrane in 2.5 mL of TBS-T with additional 0.5 pmol of Indole-modified click-competitor and 0.1 mg/ml of dextran sulphate. After incubation, the membrane was washed 3 times with 5 mL of TBS-T for 10 min while rotating. The fluorescence was visualized using an Odyssey Scanner (Li-Cor).

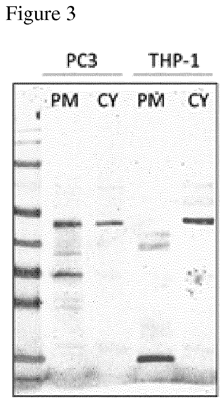

The Figure 3 shows the results of the fluorescent binding test of the monoclone of of the aptamer of SEQ ID NO: 2. The indol-functionalized Sequence (SEQ ID NO: 2), was labeled with the fluorescent dys IRD800CW. As can be taken from the Figure 3, the THP-1 cell line (THP-1, on the right) as well as the PC3 cells (on the left) with which the selection was performed, showed an intense signal in the region of band 2, thus probably the same protein.

Other cell lines tested also showed other bands that could possibly be splice variants of the target.

## Claims

1. A method of identifying or producing an aptamer, the method comprising the steps of:
a) Providing a candidate mixture of nucleic acids;
b) Contacting the candidate mixture with a target sample;
c) Partitioning nucleic acids that bind to the target sample from those that do not bind;
d) Amplifying the binding nucleic acids to produce a population of nucleic acids that bind the target sample;
e) Single strand displacement of the amplified nucleic acids,
f) optionally repeating the steps a) to e),
thereby identifying or producing an aptamer,
**characterized in that** the target sample provides one or more peptides and/or proteins in a denatured form.

2. The method according to claim 1, wherein an aptamer recognising a denatured target peptide or protein is identified or produced.

3. The method according to claim 1 or 2, wherein in step b) the target sample is provided on a Western blot.

4. The method according to any one of the preceding claims, wherein in step b) the target sample is provided as an isolated peptide or protein, particularly as a single protein band on a Western blot membrane.

5. The method according to any one of the preceding claims, wherein in step b) the target sample is provided as a multitude of peptides or proteins, preferably a fraction of peptides or proteins or a (whole) cell lysate, separated on a Western blot membrane.

6. The method according to any one of the preceding claims, wherein in step c) a single protein band or a plurality of protein bands on the Western blot membrane are selected for recovery of binding nucleic acids.

7. The method according to claim 5 or 6, wherein in step c) for an initial number of SELEX rounds a plurality of protein bands on the Western blot membrane are selected for recovery and for a following number of SELEX rounds a single protein band is selected for recovery of binding nucleic acids.

8. The method according to any one of the preceding claims, wherein in step c) the nucleic acids binding to target peptide(s) or protein(s) are recovered from the Western blot membrane using a buffer containing urea and sodium dodecyl sulfate.

9. The method according to to any one of the preceding claims, wherein in step c) the recovered nucleic acids are purified, preferably by spin column-based nucleic acid purification, phenol-chloroform extraction and/or nucleic acid precipitation.

10. The method according to any one of the preceding claims, wherein in step b) in addition to the target sample a sample for negative-selection containing denatured peptides and/or proteins is provided on the Western blot.

11. The method according to any one of the preceding claims, wherein in step a) the candidate mixture of nucleic acids comprises at least one nucleotide that is modified to comprise a functionalization introduced by click chemistry.

12. The method according to claim 11, wherein the modified nucleotide comprises an alkyne-modified nucleobase that is further modified via a 1,3 dipolar cycloaddition of an azide to yield an azide-alkyne modified nucleobase, wherein the azide preferably comprises a group selected from benzyl and indole.

13. Use of the method according to any one of the preceding claims to identify or produce an aptamer for the identification of a denatured peptide or protein.

14. The use according to claim 13, wherein the aptamer is usable in a Western blot assay.

15. An aptamer, wherein the aptamer has a nucleotide sequence selected from the group comprising:
5'-AACAAGANANACACGANGGGGGCACACCAAAGCACCGNNCGAAA-3' (SEQ ID NO: 1) and
5'-NNAACACCCACNCACGCCAANCCCACCACCCCNACACNCCCAC-3' (SEQ ID NO: 2), wherein N is selected from T or ethynyl-dU.


SEQUENCE LISTING

<110>  Rheinische Friedrich-Wilhelms Universität Bonn

<120>  A method of identifying or producing an aptamer

<130>  UD 41104/SAM

<160>  6

<170>  PatentIn version 3.3

<210>  1
<211>  44
<212>  DNA
<213>  artificial

<220>
<223>  aptamer

<220>
<221>  misc_feature
<222>  (8)..(8)
<223>  N is T or ethynyl-dU

<220>
<221>  misc_feature
<222>  (10)..(10)
<223>  N is T or ethynyl-dU

<220>
<221>  misc_feature
<222>  (17)..(17)
<223>  N is T or ethynyl-dU

<220>
<221>  misc_feature
<222>  (38)..(38)
<223>  N is T or ethynyl-dU

<220>
<221>  misc_feature
<222>  (39)..(39)
<223>  N is T or ethynyl-dU

<400>  1
aacaaganan acacganggg ggcacaccaa agcaccgnnc gaaa                    44

<210>  2
<211>  43
<212>  DNA
<213>  artificial

<220>
<223>  aptamer

<220>
<221>  misc_feature
<222>  (1)..(1)
<223>  N is T or ethynyl-dU

<220>
<221>  misc_feature
<222>  (2)..(2)
<223>  N is T or ethynyl-dU

<220>
<221>  misc_feature
<222>  (12)..(12)
<223>  N is T or ethynyl-dU

<220>
<221>  misc_feature
<222>  (21)..(21)
<223>  N is T or ethynyl-dU

<220>
<221>  misc_feature
<222>  (33)..(33)
<223>  N is T or ethynyl-dU

<220>
<221>  misc_feature
<222>  (38)..(38)
<223>  N is T or ethynyl-dU

<400>  2
nnaacaccca cncacgccaa ncccaccacc ccnacacncc cac                43


<210>  3
<211>  80
<212>  DNA
<213>  artificial

<220>
<223>  library


<220>
<221>  misc_feature
<222>  (19)..(62)
<223>  N is A, C, G or ethynyl-dU

<400>  3
agccacggaa gaaccagann nnnnnnnnnn nnnnnnnnnn nnnnnnnnnn nnnnnnnnnn      60

nngcagaagc gacagcaaca                                                  80


<210>  4
<211>  40
<212>  DNA
<213>  artificial

<220>
<223>  Click-competitor


<220>
<221>  misc_feature
<222>  (1)..(39)
<223>  N is A, C, G or ethynyl-dU

<400>  4
nnnnnnnnnn nnnnnnnnnn nnnnnnnnnn nnnnnnnnna                              40


<210>  5
<211>  18
<212>  DNA
<213>  artificial

<220>
<223>  forward primer

<400>  5
agccacggaa gaaccaga                                                      18


<210>  6
<211>  18
<212>  DNA
<213>  artificial

<220>
<223>  reverse primer

<400>  6
tgttgctgtc gcttctgc                                                      18